# EUROPEAN PATENT APPLICATION

(11) **EP 1 722 226 A1**
(43) Date of publication of application: **15.11.2006**
(21) Application number: 05719752.7
(22) Date of filing: 23.02.2005
(51) Int. Cl.: G01N 33/53

(54) **METHOD OF DISTINGUISHING TYPE A AND TYPE B ACUTE AORTIC DISSECTION AND ACUTE MYOCARDIAL INFARCTION AND KIT FOR DISTINGUISHMENT**

(30) Priority: 23.02.2004 JP 2004046877
(71) Applicant: Dainippon Sumitomo Pharma Co., Ltd., Osaka 541-8524 (JP)
(72) Inventor: HAZUI, Hiroshi, c/o Osaka Mishima Emergency Med., Osaka 569-1124 (JP); NISHIMOTO, Masayoshi, c/o Osaka Mishima Emergency, Takatsuki-shi, Osaka 569-1124 (JP); TAKESHITA, Hitoshi, c/o Osaka Mishima Emergency, Takatsuki-shi, Osaka 569-1124 (JP); OHKARU, Yasuhiko, c/o Dainippon Sumitomo Pharma Co, Osaka 564-0053 (JP)
(74) Representative: Duckworth, Timothy John
(86) International application number: PCT/JP2005/003437
(87) International publication number: WO 2005/080982

(57) **Abstract**

Provided is a method of distinguishing among Stanford type A acute aortic dissection, Stanford type B acute aortic dissection, and acute myocardial infarction, which are mutually similar in terms of clinical symptoms, and a kit for the distinguishment. Specifically, provided is a method of distinguishing among Stanford type A acute aortic dissection, Stanford type B acute aortic dissection, and acute myocardial infarction, which comprises detecting both D-dimer and H-FABP in blood separated from a person suspected of having acute aortic dissection and suspected of having acute myocardial infarction, and establishing the diagnosis on the basis of the concentrations detected, and a kit for the distinguishment.

## Description

### Technical Field

The present invention relates to a method of distinguishing among Stanford type A acute aortic dissection (hereinafter also referred to as type A acute aortic dissection), Stanford type B acute aortic dissection (hereinafter also referred to as type B acute aortic dissection), and acute myocardial infarction by detecting D-dimer and H-FABP (Heart-type Fatty Acid-Binding Protein) in blood. The present invention also relates to a kit for distinguishment used to perform the aforementioned method, and a commercial package comprising the kit.

### Background Art

Acute aortic dissection and acute myocardial infarction are diseases accounting for the great majority of the causes of sudden death due to cardiovascular diseases.

Both diseases are characterized by severe chest pain as the major complaint and sudden onset occasionally followed by the outcome of death in a short time; for appropriate measures and treatment, it is critically important to distinguish between the two diseases. In particular, in Stanford type A acute aortic dissection, dissociation sometimes involves the coronary artery and occludes the vessels, which in turn causes the pathologic condition of acute myocardial infarction. If a physician misdiagnoses a patient with acute aortic dissection and administers thrombolytic therapy, which is a treatment for acute myocardial infarction, the patient can die due to massive bleeding from the aorta.

However, acute aortic dissection and acute myocardial infarction share the feature of severe chest pain as the major complaint, as described above; in actual medical scenes, the two diseases are quite difficult to distinguish from each other on the basis of clinical symptoms. For this reason, an established diagnosis of each disease necessitates diagnostic imaging such as by echocardiography and contrast-enhanced CT (or MRI) examination. However, acute myocardial infarction and type A acute aortic dissection produce very high mortality rates in the acute phase (for example, in acute myocardial infarction, about 80% of deceased cases occur within 24 hours after attack, and the golden time for the treatment of this disease is considered to be within 6 hours from attack); a quicker diagnosis is demanded.

As a means of quickly and conveniently diagnosing a disease, laboratory testing with a biochemical marker in a biological sample as an index has been widely used. Accordingly, there is a strong demand for the development of a clinical marker enabling quick and highly accurate diagnosis of the disease affecting a patient experiencing an episode of chest pain, particularly a marker making it possible to highly accurately distinguishing among acute myocardial infarction, type A acute aortic dissection, and type B acute aortic dissection.

D-dimer is a biological protein, which is utilized to extensively assess the pathologic condition of hyperactivity of the blood coagulation/fibrinolytic system as a marker for hyperactivity of the secondary fibrinolytic system. Specifically, it is currently used as a marker for diagnosis, pathologic assessments, and therapeutic effect evaluation in disseminated intravascular coagulation (DIC) and various thrombotic diseases.

Also, it is known that patients with acute myocardial infarction have elevated D-dimer concentrations in blood compared with healthy subjects (for example, The American Journal of Medicine, (USA), December 1992, Vol. 93, pp. 651-657).

In CHEST (USA), May 2003, Vol. 123, No. 5, pp. 1375-1378, it is stated that the D-dimer concentration in blood has risen in patients developing Stanford type A or type B acute aortic dissection. However, this rise is similar between type A acute aortic dissection and type B acute aortic dissection, and no description or suggestion is given concerning the possibility of distinguishing between the two diseases using the marker.

Meantime, H-FABP is a biological protein, which occurs abundantly in the cytosol of the myocardium and is capable of binding to fatty acids and considered to be associated with the intracellular transportation of fatty acids.

In JP-A-Hei 4-31762 and Clinical Chemistry and Laboratory Medicine (Germany), 2000, Vol. 38, No. 3, PP. 231-238, a method of diagnosing acute myocardial infarction by detecting H-FABP in blood is described; pharmaceuticals for extracorporeal diagnosis of acute myocardial infarction based on the detection of H-FABP in blood (RAPICHECK (registered trademark in Japan) H-FABP, MARKIT (registered trademark in Japan) M H-FABP) are commercially available from Dainippon Pharmaceutical Co., Ltd.

However, in the "Journal of Japanese Society for Emergency Medicine", 2003, Vol. 6, No. 2, p. 226 (2-15-6), it is stated that patients developing Stanford type A or type B acute aortic dissection also have elevated H-FABP concentrations in blood. Therefore, it is considered to be difficult to distinguish among type A acute aortic dissection, type B acute aortic dissection, and acute myocardial infarction using the marker.

Also, none of the above-described literature documents state that Stanford type A acute aortic dissection, Stanford type B acute aortic dissection, and acute myocardial infarction can be highly accurately distinguished from each other by detecting both D-dimer and H-FABP in blood.

### Disclosure of Invention

In view of these circumstances, it is an object of the present invention to provide a method of distinguishing among Stanford type A acute aortic dissection, Stanford type B acute aortic dissection, and acute myocardial infarction by drawing blood, and detecting a biochemical marker therein, a kit for distinguishment, and a commercial package comprising the kit.

The present inventors drew blood from patients developing Stanford type A or type B acute aortic dissection and patients developing acute myocardial infarction, and measured D-dimer concentrations and H-FABP concentrations contained therein. As a result, the present inventors found that Stanford type A acute aortic dissection, Stanford type B acute aortic dissection, and acute myocardial infarction can be highly accurately distinguished from each other by using both markers in combination as an index of evaluation compared with each marker alone, and completed the present invention.

Accordingly, the present invention provides:
[1] a method of distinguishing among Stanford type A acute aortic dissection, Stanford type B acute aortic dissection, and acute myocardial infarction, which comprises detecting D-dimer and H-FABP in blood separated from a human suspected of having acute aortic dissection and suspected of having acute myocardial infarction,
[2] the method of distinguishment described in [1] above, which comprises comparing the D-dimer concentration detected in blood with a previously established D-dimer cutoff value, and comparing the H-FABP concentration detected in blood with a previously established H-FABP cutoff value,
[3] the method of distinguishment described in [2] above, which comprises:
   (a) judging that Stanford type A acute aortic dissection has developed if the D-dimer concentration is not less than the previously established D-dimer cutoff value (positive), and the H-FABP concentration is not less than the previously established H-FABP cutoff value (positive),
   (b) judging that Stanford type B acute aortic dissection has developed if the D-dimer concentration is not less than the aforementioned cutoff value (positive), and the H-FABP concentration is less than the aforementioned cutoff value (negative), and
   (c) judging that acute myocardial infarction has developed if the D-dimer concentration is less than the aforementioned cutoff value (negative), and the H-FABP concentration is not less than the aforementioned cutoff value (positive).
[4] the method of distinguishment described in [2] or [3] above, wherein the D-dimer cutoff value is a cutoff value established between an acute aortic dissection group and an acute myocardial infarction group, and the H-FABP cutoff value is a cutoff value established between a group consisting of an acute myocardial infarction group and a Stanford type A acute aortic dissection group and a Stanford type B acute aortic dissection group,
[5] the method of distinguishment described in [2] or [3] above, wherein the D-dimer cutoff value is a D-dimer reference value, and the H-FABP cutoff value is a cutoff value for evaluation of acute myocardial infarction,
[6] the method of distinguishment described in any of [1] to [5] above, wherein the human suspected of having acute aortic dissection and suspected of having acute myocardial infarction is a human having an episode of chest pain,
[7] the method of distinguishment described in any of [1] to [6] above, which comprises detecting D-dimer by an immunochemical method using an antibody that recognizes D-dimer, and detecting H-FABP by an immunochemical method using an antibody that recognizes H-FABP,
[8] the method of distinguishment described in [7] above, wherein the immunochemical method is the enzyme immunochemical method, the latex aggregation method, or the immunochromatography method,
[9] a reagent for distinguishing among Stanford type A acute aortic dissection, Stanford type B acute aortic dissection, and myocardial infarction, which comprises an antibody that recognizes D-dimer, and which is used in combination with a reagent comprising an antibody that recognizes H-FABP,
[10] a reagent for distinguishing among Stanford type A acute aortic dissection, Stanford type B acute aortic dissection, and myocardial infarction, which comprises an antibody that recognizes H-FABP, and which is used in combination with a reagent comprising an antibody that recognizes D-dimer,
[11] a kit for distinguishing among Stanford type A acute aortic dissection, Stanford type B acute aortic dissection, and myocardial infarction, which comprises a reagent comprising an antibody that recognizes D-dimer and a reagent comprising an antibody that recognizes H-FABP,
[12] a commercial package comprising the kit for distinguishment described in [11] above and a written matter on the kit, wherein the written matter and/or the package bears the statement that the kit can be used, or should be used, for the purpose of distinguishing among Stanford type A acute aortic dissection, Stanford type B acute aortic dissection, and acute myocardial infraction.

According to the above-described method of distinguishment of the present invention, it is possible to determine which disease of Stanford type A acute aortic dissection, Stanford type B acute aortic dissection, and acute myocardial infarction has developed, which diseases to date have been difficult to distinguish from each other due to the clinical symptom shared thereby (episode of chest pain), and physicians can take appropriate measures for each disease on the basis of the results of this distinguishment.

Also, by using the above-described kit for distinguishment or commercial package of the present invention, the same effect as the method of distinguishment of the present invention can be accomplished.

### Brief Description of the Drawings

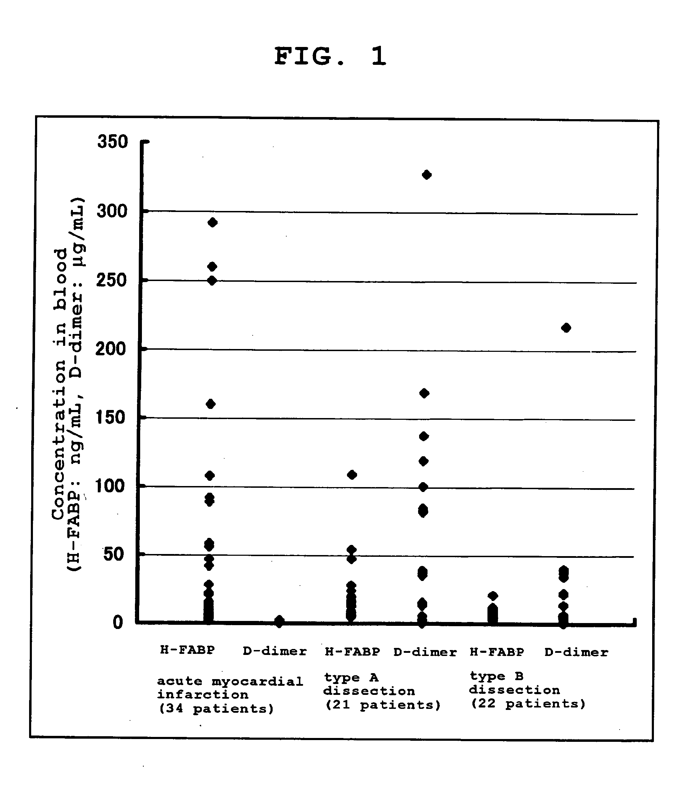

FIG. 1 shows the distribution of D-dimer concentrations and H-FABP concentrations in bloods from the patients measured in Example 1.
FIG. 2 shows an ROC curve for D-dimer generated in Example 2.
FIG. 3 shows an ROC curve for H-FABP generated in Example 3.

### Best Mode for Carrying out the Invention

The present invention relates to a method of distinguishing among Stanford type A acute aortic dissection, Stanford type B acute aortic dissection, and acute myocardial infarction by detecting D-dimer and H-FABP in blood separated from a human suspected of having acute aortic dissection and suspected of having acute myocardial infarction.

Acute aortic dissection, included in the subject diseases for the distinguishment in the present invention, is classified into either Stanford type A acute aortic dissection or Stanford type B acute aortic dissection according to Stanford's classification of disease types. Acute aortic dissection is a disease characterized by the pathologic condition wherein the tunica media, which constitutes the aortic wall, is dissociated and cleaved to the intimal side and the adventitial side. Histologically, the aortic wall consists of the tunica intima, the tunica media, and the tunica adventitia in this order from the vascular lumen side.

In acute aortic dissection, clinical symptoms and prognosis differ depending on the affected area of dissociation; classification of disease types according to the affected area is performed for deciding a therapeutic policy.

The aforementioned Stanford's classification is a representative classification of disease types; any case of acute aortic dissection is classified as either Stanford type A, in which dissociation is present in the ascending aorta from the heart to the neck, or Stanford type B, in which dissociation is not present in the ascending aorta.

The typical initial symptom of this disease is suddenly developing severe chest pain, and this symptom is generally called an episode of chest pain. The pain in an episode of chest pain may not be localized to the chest, and may involve the back to show signs of chest back pain, depending on the size of the affected area of acute aortic dissection. Note that this pain is generally understood to result from a rupture of the aortic wall.

Acute myocardial infarction, another subject disease for the distinguishment, is a disease in which the coronary artery, which controls the myocardium, is occluded and the myocardium under the control thereof necrotizes. The World Health Organization (WHO) has presented diagnostic criteria to determine that a person is developing this disease if he or she meets two or more of the requirements: A) chest pain, B) an abnormal electrocardiogram, and C) an elevation of blood levels of cardiac enzymes such as creatine kinase-MB and aspartate aminotransferase.

Acute myocardial infarction is increasing dramatically in Japan with the recent westernization of dietary life. This disease develops suddenly with the major symptoms of severe chest pain and dyspnea, and follows a very rapid course with cardiac arrest occurring simultaneously with the onset in some cases, depending on the size of the infarct lesion in the myocardium. Hence, patients often die in 1 to 2 hours after onset; making a quick and accurate diagnosis of this disease and taking appropriate measures for the disease are important to saving their lives. Severe chest pain, a symptom of acute myocardial infarction, is also called an episode of chest pain, as in acute aortic dissection, and may involve the back to show signs of chest back pain.

Acute aortic dissection and acute myocardial infarction are called "the two major diseases with chest pain" since they share an episode of chest pain as a characteristic clinical symptom, as described above, and these two diseases are difficult to distinguish from each other by clinical symptoms. However, if acute aortic dissection and acute myocardial infarction are suspected from an episode of chest pain in actual medical scenes, it is highly critical to determine which of the diseases has developed, and whether the acute aortic dissection is Stanford type A or type B, so as to ensure appropriate treatment.

Accordingly, in another mode of embodiment of the present invention, "a human having an episode of chest pain" is deemed to be "a human suspected of having acute aortic dissection and suspected of having acute myocardial infarction" and is subjected to the detection of D-dimer and H-FABP in blood.

As used herein, "a human having an episode of chest pain" means not only a human who had an episode of chest pain before blood separation, but also a human who is having an episode of chest pain at the time of blood separation.

As used herein, "distinguishment" means not only determining which one of diseases of Stanford type A acute aortic dissection, Stanford type B acute aortic dissection, and acute myocardial infarction has developed, but also estimating the extent of progression of the pathologic condition (size of lesion) of the disease evaluated, if an evaluation is made, on the basis of the D-dimer concentration and H-FABP concentration detected.

In the present invention, the detection of D-dimer in blood separated from a human is not subject to limitation; for example, the detection can be performed by any of immunochemical methods, various chromatographies represented by HPLC, and the like. In particular, an immunochemical method utilizing an antibody that recognizes D-dimer (hereinafter also referred to as anti-D-dimer antibody) is preferably used to perform the detection. If the extent of progression of each disease or the course thereof is to be determined, a method enabling the quantitative detection of D-dimer level is preferred.

The immunochemical method used to detect D-dimer in blood is not subject to limitation; as examples, conventionally known methods such as enzyme immunoassay (EIA method), the latex aggregation method, the immunochromatography method, radioimmunoassay (RIA method), fluorescence immunoassay (FIA method), luminescence immunoassay, spin immunoassay, nephelometry for judging the turbidity resulting from the formation of antigen-antibody complex, the enzyme sensor electrode method for detecting potential changes by the binding with an antigen using an antibody-immobilized membrane electrode, immune electrophoresis, Western blotting, and the like can be mentioned. Of these, the EIA method, the latex aggregation method or the immunochromatography method is preferably used to detect D-dimer.

The EIA method includes the competitive method, wherein an enzyme-labeled antigen and an antigen in the sample are allowed to compete with each other, and the non-competitive method, which does not involve such competition; of these methods, the sandwich enzyme-linked immunosorbent assay (sandwich ELISA method), a kind of non-competitive method using two kinds of antibodies, especially monoclonal antibodies, is particularly preferable with respect to specificity for antigen (D-dimer) and the ease of detection operation.

According to the sandwich ELISA method, D-dimer can be detected by putting (sandwiching) the D-dimer between two kinds of antibodies that recognize different epitopes present in the D-dimer, i.e., between a solid-immobilized anti-D-dimer antibody and an enzyme-labeled anti-D-dimer antibody. Hence, D-dimer can be detected by measuring the enzyme amount in the labeled antibody bound to the D-dimer captured by the solid-immobilized antibody.

As a kind of the sandwich ELISA method, a method utilizing the avidin-biotin reaction is also available. According to this method, it is possible to detect D-dimer in the same manner as described above, by capturing the D-dimer in blood with a solid-immobilized anti-D-dimer antibody, allowing an antigen-antibody reaction between the captured D-dimer and a biotin-labeled anti-D-dimer antibody, and then adding enzyme-labeled streptoavidin.

The latex aggregation method is an immunochemical method utilizing the aggregation reaction between antibody-sensitized latex particles and an antigen. Detection of D-dimer by this method can be performed by allowing an immune reaction between the anti-D-dimer antibody-sensitized latex particles and the D-dimer in the sample blood, and measuring the extent of the resulting aggregation of the latex particles.

In addition, the immunochromatography method is a method wherein the whole immunochemical reaction system is carried on a sheet-like carrier and the operation is completed only with the addition of blood. The outline of the detection of D-dimer by this method is as follows. First, when the sample blood is added dropwise to the carrier, an immune reaction takes place between the D-dimer in the blood and an anti-D-dimer antibody labeled with a label substance (gold colloid and the like) placed on the carrier, resulting in the formation of an immune complex. This complex develops over the carrier; when it is captured by an anti-D-dimer antibody that recognizes another epitope immobilized on a particular point of the carrier, the label substance accumulates; by macroscopically observing the extent of this accumulation, D-dimer can be detected.

In performing the immunochromatography method, no special measuring instrument is required; this method is advantageous in cases where an evaluation outside the hospital or an immediate evaluation in emergencies and the like is desired. This method is suitable for the qualitative detection of the presence of D-dimer at a given level or higher; it is easily possible to have settings so that a positive result will be obtained if D-dimer is detected at a level not less than the cutoff value, and a negative result is obtained if D-dimer is detected at a level less than the cutoff value, provided that the cutoff value described below is established in advance.

Note that immunochemical methods intended to detect D-dimer in blood, such as the above-described sandwich ELISA method and latex aggregation method, are already known; D-dimer detection reagents utilizing these methods are commercially available, as described below. By using these reagents, detection of D-dimer in blood separated from a human can easily be performed.

Meantime, detection of H-FABP in blood can be performed in the same manner as the method of detecting D-dimer described above.

Of these detection methods, it is preferable to detect H-FABP by an immunochemical method using an antibody that recognizes H-FABP (hereinafter also referred to as anti-H-FABP antibody) as in the case of detection of D-dimer; in particular, the EIA method, the latex aggregation method or the immunochromatography method is preferably used to detect H-FABP.

Note that immunochemical methods intended to detect H-FABP in blood, such as the sandwich ELISA method and the immunochromatography method, are already known, as described below; H-FABP detection reagents utilizing these methods are commercially available. By using these reagents, detection of H-FABP in blood separated from a human can easily be performed.

In the present invention, the blood used as a sample may be any of whole blood, serum, and plasma, and these can be obtained as appropriate by treating blood drawn from a human by a conventional method.

Distinguishment among the individual diseases can be achieved by comparing the D-dimer and H-FABP concentrations in blood detected as described above with, for example, previously calculated mean values for a large number of patients developing each disease, with the distribution map of values for each patient, and the like.

For example, the mean ± SD (standard deviation) of D-dimer concentration and mean ± SD of H-FABP concentration in blood from 21 patients with type A acute aortic dissection described in Examples below were 62.3 ± 78.7 µg/mL and 21.0 ± 24.0 ng/mL, respectively, and those in 22 patients with type B acute aortic dissection were 22.0 ± 45.6 µg/mL and 6.54 ± 4.12 ng/mL, respectively. The mean ± SD of D-dimer concentration in blood from 34 patients with acute myocardial infarction was 0.51 ± 0.56 µg/mL, and that of H-FABP concentration was 51.8 ± 77.8 ng/mL. Hence, both D-dimer concentration and H-FABP concentration varied widely among the individual diseases.

In another mode of embodiment of the present invention, the individual diseases can be distinguished from each other by previously establishing a D-dimer cutoff value and an H-FABP cutoff value, and comparing the detected D-dimer concentration and H-FABP concentration in blood with these cutoff values. For example, it can be judged that:
(a) Stanford type A acute aortic dissection is likely to have developed if the detected D-dimer concentration in blood is not less than the aforementioned cutoff value (positive), and the H-FABP concentration is not less than the aforementioned cutoff value (positive),
(b) Stanford type B acute aortic dissection is likely to have developed if the D-dimer concentration is not less than the aforementioned cutoff value (positive), and the H-FABP concentration is less than the aforementioned cutoff value (negative), and
(c) acute myocardial infarction is likely to have developed if the D-dimer concentration is less than the aforementioned cutoff value (negative), and the H-FABP concentration is not less than the aforementioned cutoff value (positive).

It is possible to highly accurately determine which of Stanford type A acute aortic dissection, Stanford type B acute aortic dissection, and acute myocardial infarction has developed in a human suspected of having acute aortic dissection and suspected of having acute myocardial infarction, by adopting the evaluation criteria (a) to (c) above, as described in Examples below.

"A cutoff value" is a value that meets the requirements for both high diagnostic sensitivity (true positive rate) and high diagnostic specificity (true negative rate) when a disease is evaluated on the basis of the value as the criterion. For D-dimer, the D-dimer concentration in blood, which gives high positive rates (not less than the cutoff value) in patients developing acute aortic dissection and gives high negative rates (less than the cutoff value) in patients developing acute myocardial infarction, can be established as a cutoff value. For H-FABP, for example, the H-FABP concentration in blood, which gives high positive rates in patients who developing acute myocardial infarction and patients developing Stanford type A acute aortic dissection and gives high negative rates in patients developing Stanford type B acute aortic dissection, can be established as a cutoff value.

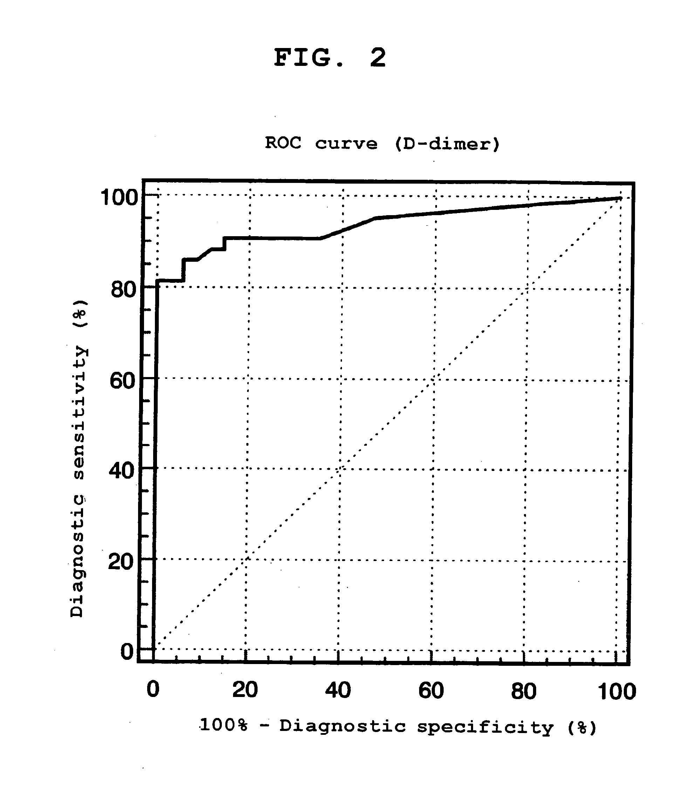

The method of calculating a cutoff value is well known in the relevant field. In the case of D-dimer, for example, D-dimer in blood is detected in a large number of patients with acute aortic dissection and patients with acute myocardial infarction, the diagnostic sensitivity and diagnostic specificity at each concentration detected are determined, and an ROC (Receiver Operating Characteristic) curve is generated on the basis of these values using a commercially available analytical software program (see FIG. 2). Then, the D-dimer concentration for a diagnostic sensitivity and diagnostic specificity as close to 100% as possible is determined, and this value can be used as the cutoff value. Note that, for example, it is possible to determine the diagnostic efficiency (ratio of the sum of the number of true positive cases and the number of true negative cases to the total number of all cases examined) at each detected concentration, and use the concentration for the highest diagnostic efficiency as a cutoff value. In an Example below, the D-dimer cutoff value established between an acute aortic dissection group and an acute myocardial infarction group was 2.4 µg/mL.

An H-FABP cutoff value can be determined by detecting H-FABP in blood from a large number of patients with acute myocardial infarction and patients with acute aortic dissection, and following the same procedure as in the case of D-dimer. In an Example below, the H-FABP cutoff value established between a group consisting of an acute myocardial infarction group and a Stanford type A acute aortic dissection group and a Stanford type B acute aortic dissection group was 6.7 ng/mL.

As a D-dimer cutoff value, a known reference value adopted in ordinary testing for abnormalities of the blood coagulation/fibrinolytic system can also be used. The reference value indicates the upper limit (for example, the upper limit of 95% interval) of a range in which the D-dimer concentrations in blood from the majority of healthy subjects fall; if the D-dimer concentration in blood exceeds this value, it is judged that there is possibly an abnormality in the blood coagulation/fibrinolytic system. For the reference value as such, a particular value has been established for each reagent used; the reference value of D-dimer in blood for the reagent used in an Example below is 400 ng/mL.

As an H-FABP cutoff value, a known cutoff value determined to evaluate acute myocardial infarction can also be used. For example, in "Clinical Chemistry and Laboratory Medicine", (2000), 38(3), PP. 231-238, the H-FABP assay reagent described in an Example below is used, and the cutoff value for evaluation of acute myocardial infarction determined therein was 6.2 ng/mL.

Note that even when the same sample is analyzed, the measured values of D-dimer and H-FABP may differ depending on the measuring method and reagent used; therefore, the cutoff values must be established for each measuring method and reagent used.

When performing the method of distinguishment of the present invention, the distinguishment among individual diseases can be made more accurate by combining the method with other methods which are known ordinary tests for cardiovascular diseases, for example, chest plain roentgenography, ultrasonic tomography, CT scanning and the like.

The D-dimer detection regent of the present invention, which comprises an antibody that recognizes D-dimer, can be suitably used in cases where D-dimer is detected by an immunochemical method in performing the method of distinguishment of the present invention. Accordingly, the present invention also provides a reagent for distinguishing among Stanford type A acute aortic dissection, Stanford type B acute aortic dissection, and acute myocardial infarction, which comprises an antibody that recognizes D-dimer. The reagent is characterized by being used in combination with an H-FABP detection reagent (specifically, for example, an antibody that recognizes H-FABP, and the like).

The anti-D-dimer antibody contained in the aforementioned distinguishment reagent may be any of a polyclonal antibody and a monoclonal antibody, as long as it recognizes a D-dimer present in blood separated from a human and has specific immunoreactivity to D-dimer. Of the two types of antibodies, a monoclonal antibody is preferred in terms of stable supply of antibody, and also in terms of high specificity for D-dimer and homogeneity.

Such an anti-D-dimer antibody can be produced by a known means, and is contained in the aforementioned distinguishment reagent in a free state, in a labeled state, or in a solid-immobilized state.

A polyclonal antibody can be produced by immunizing an animal such as a mouse, rat, or rabbit with D-dimer separated and purified from human blood by a conventional method, along with an appropriate adjuvant, drawing blood, and subjecting the blood to a known treatment.

A monoclonal antibody can be produced by collecting splenocytes from an animal immunized as described above, fusing the splenocytes with myeloma cells by the method of Milstein et al., performing antibody-producing cell screening and cloning and the like, establishing a cell line that produces an anti-D-dimer antibody, and culturing the cell line. Here, the D-dimer used as an immunizing antigen is not necessarily be a naturally occurring D-dimer present in human blood, and may be a recombinant D-dimer obtained by a gene engineering technique or an equivalent thereto (fragment) having the same effect.

When the sandwich ELISA method is adopted for the D-dimer detection reagent, the thus-obtained anti-D-dimer antibody is contained in the reagent in the form of a solid-immobilized anti-D-dimer antibody and an enzyme-labeled anti-D-dimer antibody.

A solid-immobilized anti-D-dimer antibody can be produced by binding an anti-D-dimer antibody obtained as described above to a solid phase (for example, microplate wells and plastic beads). Binding to the solid phase can be achieved usually by dissolving the antibody in an appropriate buffer such as a citrate buffer, and contacting the solid phase surface and the antibody solution for an appropriate time (1 to 2 days).

Furthermore, it is common practice to contact a phosphate buffer solution of bovine serum albumin (BSA), bovine milk protein or the like with the solid phase, and block the solid phase surface portion not coated by the antibody with the aforementioned BSA, bovine milk protein or the like, so as to suppress non-specific adsorption and non-specific reactions.

An enzyme-labeled anti-D-dimer antibody can be produced by binding (labeling) an anti-D-dimer antibody that recognizes an epitope different from that recognized by the above-described solid-immobilized antibody and an enzyme. As the enzyme for labeling the antibody, alkaline phosphatase, glucose oxidase, peroxidase, β-galactosidase and the like can be mentioned. Binding of these enzymes and an anti-D-dimer antibody can be achieved by a method known per se, for example, the glutaraldehyde method, the maleimide method and the like.

When the avidin-biotin reaction is utilized in the ELISA method, the biotin-labeled anti-D-dimer antibody contained in the D-dimer detection reagent can be produced by using, for example, a commercially available biotin-labeling kit.

When performing the sandwich ELISA method, a standard substance, a washing solution, enzyme activity determination reagents (substrate, substrate solvent, reaction stop solution and the like), enzyme-labeled streptoavidin (in cases where the avidin-biotin reaction is utilized) and the like are used as necessary, in addition to the aforementioned anti-D-dimer antibody. Therefore, the D-dimer detection reagent may be in the form of a kit comprising these as constituent reagents, in addition to the anti-D-dimer antibody.

As the aforementioned substrate, an appropriate one is chosen according to the labeling enzyme selected. For example, when peroxidase is selected as the enzyme, o-phenylenediamine (OPD), tetramethylbenzidine (TMB) and the like are used; when alkaline phosphatase is selected, p-nitrophenyl phosphate (PNPP) and the like are used. For the reaction stop solution and substrate solvent, conventionally known ones can be used as appropriate without limitation according to the enzyme selected.

Note that a large number of D-dimer detection reagents based on the sandwich ELISA method are commercially available (for example, Boehringer Mannheim "Asserachrom D-dimer" (trade name), Fujirebio "Dimer Test EIA" (trade name) and the like), and these reagents can also be used as the aforementioned detection reagent of the present invention.

When the latex aggregation method is adopted for the D-dimer detection reagent, the anti-D-dimer antibody is contained in the reagent in the form of a latex-sensitized anti-D-dimer antibody. Sensitization (binding) of an anti-D-dimer antibody to latex particles can be achieved by a method known in the relevant field, for example, the chemical bonding method (a method using carbodiimide, glutaraldehyde or the like as a crosslinking agent) and the physical adsorption method.

When performing the latex aggregation method, a dilution/stabilization buffer solution, a standard antigen and the like are used as necessary, in addition to the above-described latex-sensitized antibody. Therefore, the D-dimer detection reagent may be in the form of a kit comprising these as constituent reagents, in addition to the anti-D-dimer antibody.

Note that a large number of D-dimer detection reagents based on the latex aggregation method are commercially available (for example, DIA-IATRON "LPIA ACE D-D-dimer" (trade name), Nippon Roche "COAGUSOL D-dimer" (trade name) and the like), and these reagents can also be used as the aforementioned detection reagent of the present invention.

When the immunochromatography method is adopted for the D-dimer detection reagent, the anti-D-dimer antibody is contained in the reagent in the form of a solid-immobilized anti-D-dimer antibody and a labeled anti-D-dimer antibody. As the label substance in the labeled anti-D-dimer antibody, ones known in the relevant field can be used as appropriate, but gold colloid out of them is preferably used.

The D-dimer detection reagent of the present invention based on such immunochromatography method can be produced with reference to a known method, for example, "Clinical Biochemistry", (2001), 34, pp. 257-263, which describes a method of producing an H-FABP detection reagent based on the immunochromatography method described below.

The H-FABP detection regent of the present invention, which comprises an antibody that recognizes H-FABP, can be suitably used in cases where H-FABP is detected by an immunochemical method in performing the method of distinguishment of the present invention. Accordingly, the present invention also provides a reagent for distinguishing among Stanford type A acute aortic dissection, Stanford type B acute aortic dissection, and acute myocardial infarction, which comprises an antibody that recognizes H-FABP. The reagent is characterized by being used in combination with a D-dimer detection reagent (specifically, for example, an antibody that recognizes D-dimer, and the like).

Although the anti-H-FABP antibody contained in the aforementioned distinguishment reagent may be any of a polyclonal antibody and a monoclonal antibody, as long as it recognizes an H-FABP present in blood separated from a human and has specific immunoreactivity to an H-FABP, a monoclonal antibody is preferred for the same reason as with anti-D-dimer antibody. Such an anti-H-FABP antibody can be produced by a known means. The aforementioned distinguishment regent of the present invention, which comprises an antibody that recognizes H-FABP, can also be produced in the same manner as with the distinguishment reagent comprising an anti-D-dimer antibody described above, and these production methods are also already known.

For example, a specific method of producing an H-FABP detection reagent based on the sandwich ELISA method is described in the "Journal of Immunological Methods", (1995), 178, pp. 99-111; a detection reagent based on the latex aggregation method is described in "Clinical Chemistry", (1998), 44, No. 7, pp. 1564-1567 and JP-A-Hei 4-31762; a detection reagent based on the immunochromatography method is described in the aforementioned "Clinical Biochemistry", (2001), 34, pp. 257-263.

Also, detection reagents for H-FABP in blood are commercially available from Dainippon Pharmaceutical Co., Ltd. (immunochromatography reagent "RAPICHECK (registered trademark in Japan) H-FABP" and sandwich ELISA reagent "MARKIT (registered trademark in Japan) M H-FABP"), and these reagents can be used as the aforementioned detection reagents of the present invention.

The present invention relates to a kit for distinguishing among Stanford type A acute aortic dissection, Stanford type B acute aortic dissection, and acute myocardial infarction, which comprises the aforementioned D-dimer detection reagent and the aforementioned H-FABP detection reagent.

The distinguishment kit of the present invention can be suitably used in cases where the above-described diseases are distinguished from each other by detecting D-dimer and H-FABP in blood by an immunochemical method, and is intended to accomplish the same object as the method of distinguishment of the present invention.

As such, the distinguishment kit of the present invention is constituted by a D-dimer detection reagent and an H-FABP detection reagent. Although these detection reagents are generally contained in the kit in mutually independent forms, the kit may be in the form of a single detection reagent that concurrently comprises an anti-D-dimer antibody and an anti-H-FABP antibody, provided that it is produced as a reagent capable of simultaneously detecting a plurality of antigens, like the reagents described in, for example, JP-A-Hei 8-5635 and JP-A-2000-292427.

For example, the aforementioned single detection reagent adopting the sandwich ELISA method comprises an anti-D-dimer antibody and anti-H-FABP antibody immobilized on the same well on a microplate, and an anti-D-dimer antibody and anti-H-FABP antibody labeled with mutually separately detectable enzymes.

The distinguishment kit of the present invention is supplied in the form of a commercial package in actual medical scenes. Such a commercial package comprises the distinguishment kit of the present invention and a written matter concerning the kit (what is called "package insert"), and the aforementioned written matter and/or the aforementioned commercial package bears the statement that the distinguishment kit of the present invention can be used, or should be used, for the purpose of distinguishing among Stanford type A acute aortic dissection, Stanford type B acute aortic dissection, and acute myocardial infarction.

### Examples

The present invention is hereinafter described in more detail by means of the following Examples.

### Example 1: Measurement of D-dimer and H-FABP in blood from patients with Stanford type A acute aortic dissection, Stanford type B acute aortic dissection, and acute myocardial infarction

Of patients who visited a hospital with a complaint of chest pain (including chest back pain), those having the established diagnosis of acute aortic dissection (43 patients in total, consisting of 21 patients with Stanford type A and 22 patients with Stanford type B) and those having the established diagnosis of acute myocardial infarction (34 patients) had their blood drawn at the time of visit, and the D-dimer concentrations and H-FABP concentrations in the blood samples were measured as described below.

Note that informed consent was obtained from these patients.

### (1) Measurement of D-dimer

D-dimer concentrations were measured using "STA Liatest D-dimer" (trade name, manufactured by Roche Diagnostics, Inc.), a D-dimer assay reagent adopting the latex aggregation method as the measurement principle, as described below. Note that the reference value for this reagent has been established at 400 ng/mL.

1.8 mL of whole blood was drawn from a vein of each patient into a commercially available sodium citrate blood drawing tube (INSE-PACK-C for coagulation testing [Sekisui Chemical Co., Ltd.], 0.2 mL of 3.13% sodium citrate contained). Subsequently, the blood drawing tube was gently inverted to mix the contents, and centrifuged at 3000 rpm and room temperature for 5 minutes. After centrifugation, the blood drawing tube as is was set to the automated D-dimer measuring equipment STA (manufactured by Roche Diagnostics, Inc.) and analyzed. D-dimer concentrations were measured using the above-described assay reagent. Regarding analytical conditions, 50 µL of blood sample volume, 100 µL of buffer solution (reagent 1 attached to the above-described D-dimer detection reagent; 11.5 mg/mL tris(hydroxymethyl)aminomethane, 1 mg/mL sodium azide, 23.5 mg/mL NaCl, 0.95 mg/mL methyl para-oxybenzoate [antiseptic]), 50 µL of diluent for STA instrument for the above-described D-dimer assay reagent, and 150 µL of latex-sensitized anti-D-dimer antibody solution (reagent 2 attached to the above-described D-dimer detection reagent; 55 µg/mL anti-human D-dimer mouse monoclonal antibody, 0.65 mg/mL latex, 3 mg/mL bovine serum albumin, 0.95 mg/mL methyl para-oxybenzoate [antiseptic], 1 mg/mL sodium azide) were automatically added, and a measurement was performed with an analytical time of 240 seconds and a reaction temperature of 37°C. The calibration curve range for this assay reagent is 0.2 to 4.0 µg/mL of D-dimer concentration; samples showing high values exceeding this concentration range were subjected to reanalysis. The samples with high values were diluted with the aforementioned diluent for STA instrument at a pre-defined dilution rate in advance, and again applied to the STA equipment to measure the D-dimer concentration in the sample. The measured D-dimer values were computer-processed and managed on line.

### (2) Measurement of H-FABP

Blood drawn in the same manner as (1) above was centrifuged at 3000 rpm to obtain a plasma fraction. This plasma fraction was used as the sample for ELISA for H-FABP measurement. The ELISA for H-FABP measurement used was commercially available "MARKIT M H-FABP" (Dainippon Pharmaceutical Co., Ltd.). 70 µL of kit buffer solution was added to the sample dispensing plate attached to this ELISA kit in advance, 70 µL of the previously obtained serum or plasma was added thereto, and the mixture was stirred, after which 100 µL thereof was added to an antibody-coupled plate having an anti-human H-FABP monoclonal antibody immobilized thereon, and the plate was allowed to stand at 25°C for 30 minutes to react the H-FABP in the sample with the antibody. After each well was washed three times with 300 µL of the washing solution attached to the above-described kit, a horseradish-peroxidase-labeled anti-human H-FABP monoclonal antibody was added, and the plate was allowed to stand at 25°C for 30 minutes to react the labeled antibody to the H-FABP captured by the immobilized antibody. After each well was washed in the same manner as described above to remove the excess enzyme-labeled antibody, 100 µL of a substrate solution (o-phenylenediamine/hydrogen peroxide) was added to each well, and the enzyme reaction was performed at 25°C for 15 minutes. Subsequently, 100 µL of a reaction stop solution was added. After stirring, the absorbance of each well was determined at a main wavelength of 492 nm (side wavelength 620 nm). A standard curve was generated on the basis of absorbance values obtained with similarly assayed standard H-FABP solutions, and the H-FABP concentration of the sample was obtained.

### (3) Measurement results

The measurement results are shown in Table 1 below; measured D-dimer values and measured H-FABP values differed widely among the individual diseases.

The groups of subjects were compared by Mann-Whitney U-test. As a result, for D-dimer, significant differences were observed at a significance level of 1% between the acute myocardial infarction group (AMI), and the type A acute aortic dissection group (A-AAD) and the type B acute aortic dissection group (B-AAD), and a significant difference was observed at a significance level of 5% between the type A acute aortic dissection group and the type B acute aortic dissection group.

For H-FABP, a significant difference was observed at a significance level of 1% between the acute myocardial infarction group and the type B acute aortic dissection group, and between the type A acute aortic dissection group and the type B acute aortic dissection group.

**Table 1**

| D-dimer and H-FABP concentrations in blood from patients with individual diseases | | | |
|---|---|---|---|
| Patients (name of disease) | Number of patients | Concentrations in blood (mean±standard deviation) | |
| | | D-dimer (µg/mL) | H-FABP (ng/mL) |
| Type A acute aortic dissection | 21 | 62.3±78.7 | 21.0±24.0 |
| Type B acute aortic dissection | 22 | 22.0±45.6 | 6.54±4.12 |
| Acute myocardial infarction (AMI) | 34 | 0.51±0.56 | 51.8±77.8 |

| Mann-Whitney U-test: | | |
|---|---|---|
| D-dimer; | AMI v. A-AAD | p<0.001 |
| | AMI v. B-AAD | p<0.001 |
| | A-AAD v. B-AAD | p=0.0389 |
| H-FABP; | AMI v. A-AAD | p=0.7816 |
| | AMI v. B-AAD | p<0.001 |
| | A-AAD v. B-AAD | p=0.00117 |

### Example 2: Distinguishment among individual diseases - 1

### (1) Establishing a D-dimer cutoff value

For the measured values obtained in Example 1, ROC curve analysis was performed using the ROC analytical software program "MedCalc" (trade name, Med Calc Software Company [Belgium]), and a D-dimer cutoff value that makes it possible to distinguish between the acute aortic dissection group and the acute myocardial infarction group was established. The value calculated from the ROC curve (FIG. 2) was 2.4 µg/mL.

### (2) Establishing an H-FABP cutoff value

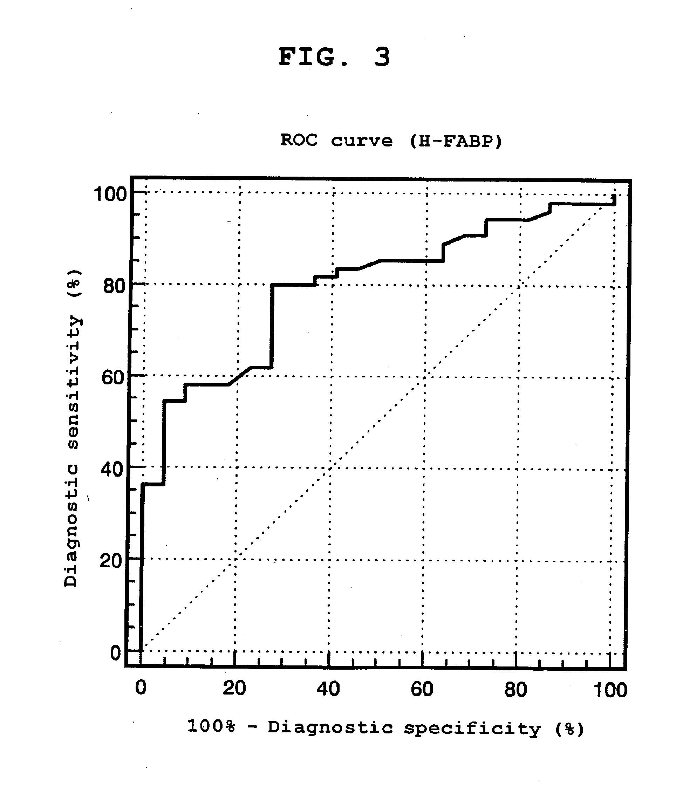

For the measured values obtained in Example 1, an H-FABP cutoff value that makes it possible to distinguish between the group consisting of the acute myocardial infarction group and the Stanford type A acute aortic dissection group and the Stanford type B acute aortic dissection group was established. The value calculated from the ROC curve (FIG. 3) was 6.7 ng/mL.

### (3) Calculation of diagnostic efficiency

Diagnostic efficiency was calculated from the measured values obtained in Example 1 using the cutoff values obtained in (1) and (2) above as the criteria, and a comparison was made with cases of each marker used alone.

"Diagnostic efficiency" is the ratio of the sum of the number of true positive cases and the number of true negative cases to the total number of all cases examined As the diagnostic efficiency value increases, the diagnostic criteria specified for diagnosing a disease are capable of more accurately diagnosing the disease. Diagnostic efficiency is calculated on the basis of diagnostic sensitivity (true positive rate) and diagnostic specificity (true negative rate).

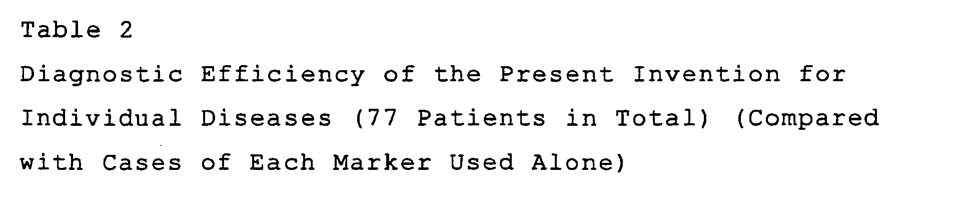

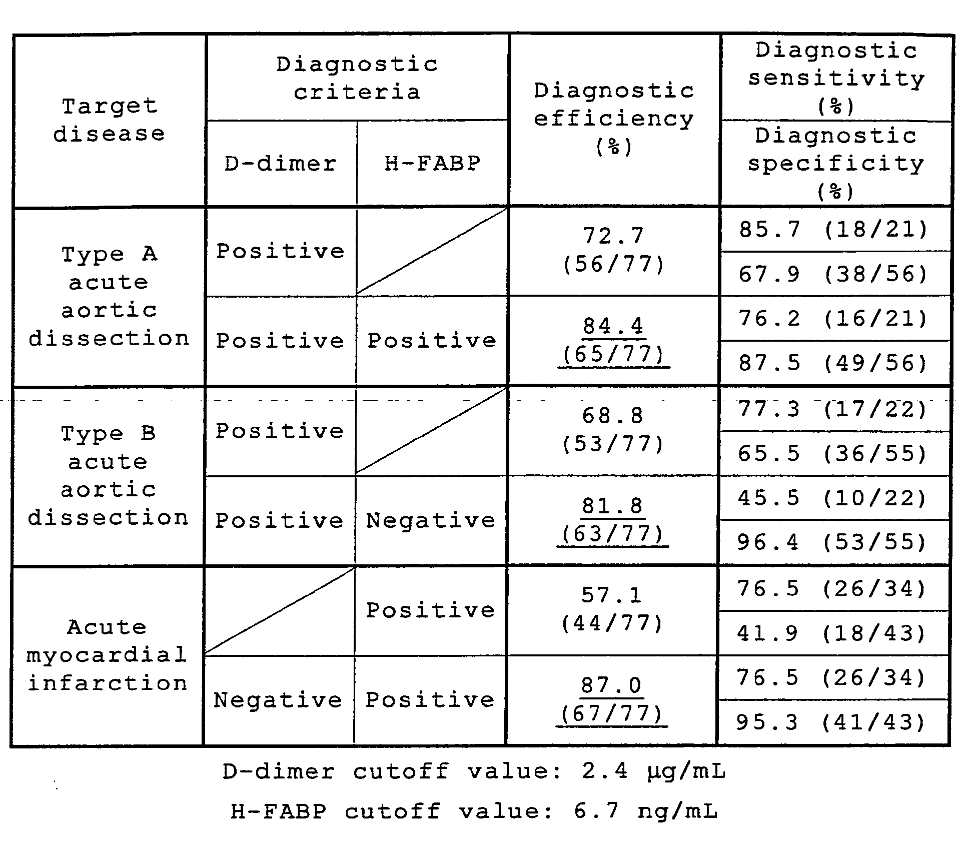

As shown in Table 2 above, the diagnostic efficiency in the case of D-dimer alone was 72.7% for type A acute aortic dissection and 68.8% for type B acute aortic dissection. The diagnostic efficiency in the case of H-FABP alone was 57.1% for acute myocardial infarction.

Meantime, the diagnostic efficiency in the case of the two markers concurrently used as the diagnostic criteria was very high at 84.4%, 81.8%, and 87.0%, respectively; for all target diseases, the diagnostic efficiency improved remarkably compared with the cases of each marker used alone.

### Example 3: Distinguishment among individual diseases - 2

Diagnostic efficiency for each disease was calculated in the same manner as Example 2 using the reference value (400 ng/mL) for the reagent used in Example 1 as the D-dimer cutoff value and the cutoff value (6.2 ng/mL) for evaluating acute myocardial infarction established for the reagent used in Example 1 as the H-FABP cutoff value, and a comparison was made with the cases of each marker used alone.

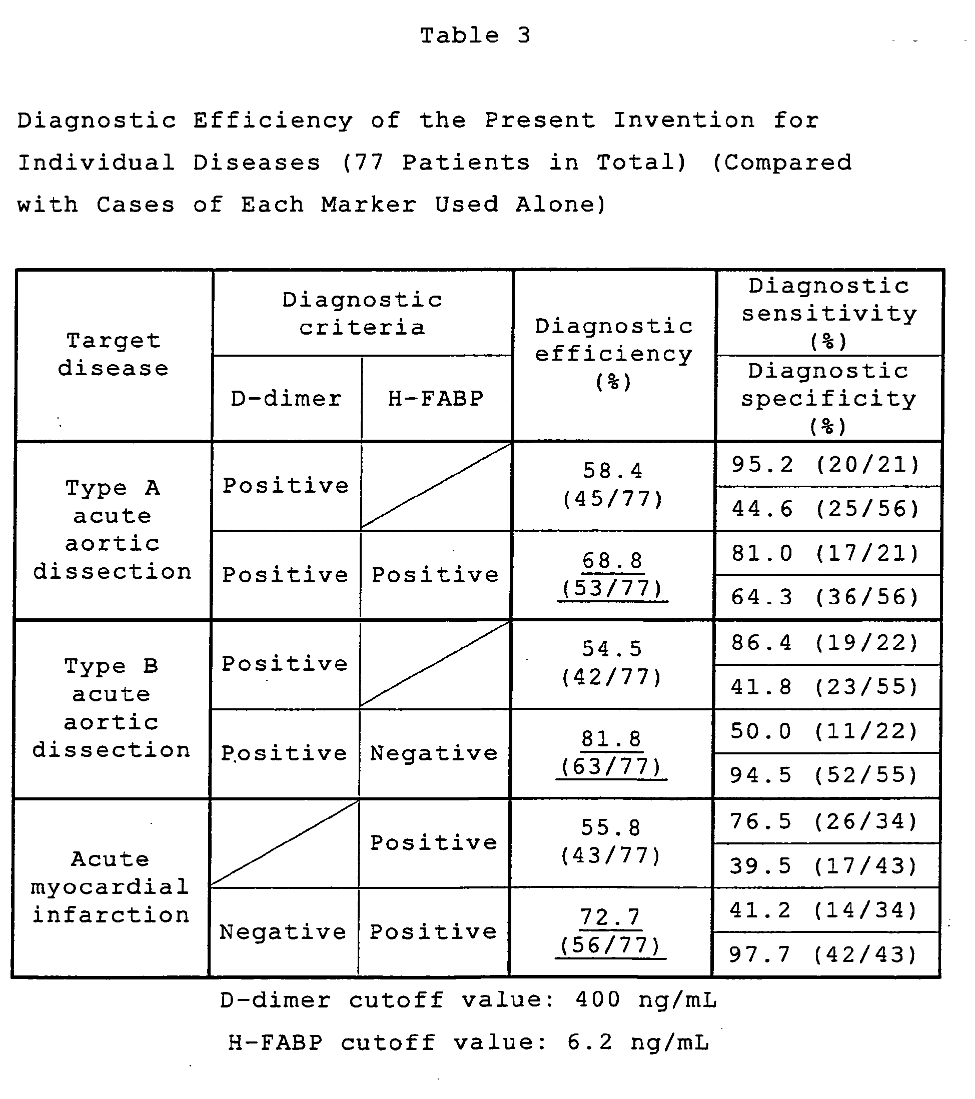

As shown in Table 3 above, the diagnostic efficiency in the case of D-dimer alone was 58.4% for type A acute aortic dissection and 54.5% for type B acute aortic dissection. The diagnostic efficiency in the case of H-FABP alone was 55.8% for acute myocardial infarction.

Meantime, the diagnostic efficiency in the case of two markers concurrently used as the diagnostic criteria was very high at 68.8%, 81.8%, and 72.7%, respectively; for all target diseases, the diagnostic efficiency improved remarkably compared with the cases of each marker used alone.

### Industrial Applicability

By using the method of distinguishment and kit for distinguishment of the present invention, it is possible to highly accurately distinguish among Stanford type A acute aortic dissection, Stanford type B acute aortic dissection, and acute myocardial infarction, which to date have been difficult to distinguish from each other on the basis of the clinical symptoms thereof. Physicians can take appropriate measures for each disease on the basis of the results of this distinguishment.

This application is based on a patent application No. 2004-046877 filed in Japan (filing date: February 23, 2004), the contents of which are incorporated in full herein by this reference.

## Claims

1. A method of distinguishing among Stanford type A acute aortic dissection, Stanford type B acute aortic dissection, and acute myocardial infarction, which comprises detecting D-dimer and H-FABP in blood separated from a human suspected of having acute aortic dissection and suspected of having acute myocardial infarction.

2. The method of distinguishment of claim 1, which comprises comparing the D-dimer concentration detected in blood with a previously established D-dimer cutoff value, and comparing the H-FABP concentration detected in blood with a previously established H-FABP cutoff value.

3. The method of distinguishment of claim 2, which comprises:
(a) judging that Stanford type A acute aortic dissection has developed if the D-dimer concentration is not less than the previously established D-dimer cutoff value, and the H-FABP concentration is not less than the previously established H-FABP cutoff value,
(b) judging that Stanford type B acute aortic dissection has developed if the D-dimer concentration is not less than the aforementioned cutoff value, and the H-FABP concentration is less than the aforementioned cutoff value, and
(c) judging that acute myocardial infarction has developed if the D-dimer concentration is less than the aforementioned cutoff value, and the H-FABP concentration is not less than the aforementioned cutoff value.

4. The method of distinguishment of claim 2 or 3, wherein the D-dimer cutoff value is a cutoff value established between an acute aortic dissection group and an acute myocardial infarction group, and the H-FABP cutoff value is a cutoff value established between a group consisting of an acute myocardial infarction group and a Stanford type A acute aortic dissection group and a Stanford type B acute aortic dissection group.

5. The method of distinguishment of claim 2 or 3, wherein the D-dimer cutoff value is a D-dimer reference value, and the H-FABP cutoff value is a cutoff value for evaluation of acute myocardial infarction.

6. The method of distinguishment of any one of claims 1 to 5, wherein the human suspected of having acute aortic dissection and suspected of having acute myocardial infarction is a human having an episode of chest pain.

7. The method of distinguishment of any one of claims 1 to 6, which comprises detecting D-dimer by an immunochemical method using an antibody that recognizes D-dimer, and detecting H-FABP by an immunochemical method using an antibody that recognizes H-FABP.

8. The method of distinguishment of claim 7, wherein the immunochemical method is the enzyme immunochemical method, the latex aggregation method, or the immunochromatography method.

9. A reagent for distinguishing among Stanford type A acute aortic dissection, Stanford type B acute aortic dissection, and myocardial infarction, which comprises an antibody that recognizes D-dimer, and which is used in combination with a reagent comprising an antibody that recognizes H-FABP.

10. A reagent for distinguishing among Stanford type A acute aortic dissection, Stanford type B acute aortic dissection, and myocardial infarction, which comprises an antibody that recognizes H-FABP, and which is used in combination with a reagent comprising an antibody that recognizes D-dimer.

11. A kit for distinguishing among Stanford type A acute aortic dissection, Stanford type B acute aortic dissection, and myocardial infarction, which comprises a reagent comprising an antibody that recognizes D-dimer and a reagent comprising an antibody that recognizes H-FABP.

12. A commercial package comprising the kit for distinguishment of claim 11 and a written matter on the kit, wherein the written matter and/or the package bears the statement that the kit can be used, or should be used, for the purpose of distinguishing among Stanford type A acute aortic dissection, Stanford type B acute aortic dissection, and acute myocardial infarction.
